# EUROPEAN PATENT APPLICATION

(11) **EP 4 797 275 A1**
(43) Date of publication of application: **26.08.2026**
(21) Application number: 26160571.1
(22) Date of filing: 25.02.2026
(51) Int. Cl.: G16H 20/60, G16H 40/63, G16H 50/20, A61B 5/00, A61M 5/142, A61M 5/172

(54) **MEDICATION DELIVERY SYSTEM**

(30) Priority: 25.02.2025 US 202563763191 P
(71) Applicant: Willow Laboratories, Inc., Irvine, CA 92617 (US)
(72) Inventor: Kiani, Massi Joe E., Laguna Niguel (US)
(74) Representative: Zacco Sweden AB

(57) **Abstract**

A medication delivery system, including a wearable device configured to automatically deliver medication based on food order information received from a user computing device. The medication is delivered via a pump, and the medication may be insulin. The food order information includes timing information and/or information about the food ordered necessary to change a medication delivery. A controller determines a medication adjustment based on an estimated physiological response to the food order information. A delivery pump automatically delivers the medication, e.g., an insulin bolus, prior to or in anticipation of food consumption.

## Description

### TECHNICAL FIELD

The present disclosure generally relates to disease management systems and methods to control medication delivery.

### BACKGROUND

Diabetes is a chronic disease that impacts many individuals, both adults and children. The management of diabetes may include the measurement of glucose within the interstitial space including blood and/or interstitial fluid of a patient and administration of insulin to the patient. A closed loop insulin administration system includes both a sensor to take glucose measurements from the interstitial space including blood and/or interstitial fluid of the patient and an insulin administration device which administers insulin to the patient based on the glucose measurements. Closed loop insulin administration systems allow individuals impacted by diabetes to go about daily life with much less worry about their insulin or glucose levels which can vastly improve a diabetic's quality of life.

### SUMMARY

In one aspect, a wearable device for medication delivery is provided that includes a pump configured to deliver medication to a user and a controller having one or more processors and memory storing executable instructions. The controller can be configured to receive food order information including at least one of food delivery timing and an indication of food type, food quantity, food carbohydrate count, food calorie count, or other food-related information usable to determine a medication delivery adjustment, to determine a medication adjustment and corresponding adjustment timing based on the food order information, and to automatically deliver the medication using the pump based on the medication adjustment. In some aspects, the wearable device can include one or more sensors configured to measure at least one physiological measurement from a portion of the user. In some aspects, the medication can comprise insulin and the medication delivery includes delivery of an insulin bolus. In some aspects, the device includes a sensor disposed in the wearable device or in a separate wearable device, and the wearable device is configured to obtain a physiological measurement from the sensor. In some aspects, the wearable device can include at least one glucose sensor configured to provide glucose measurements to the controller. In some aspects, the controller can determine an estimated physiological change based on the food order information and a physiological measurement, including computing a medication adjustment based on an estimated change in blood glucose associated with the food order information. In some aspects, the estimated change in blood glucose can be determined using historical food consumption data associated with the user, statistical analysis, trend assessment, machine learning, logistic regression, and/or a lookup table. In some aspects, the food order information can further include ingredient information. In some aspects, the controller can calculate a medication dose value and administers medication by actuating the pump and operates the pump according to a closed-loop medication delivery protocol. In some aspects, the pump can be fluidly coupled to a medication bladder and a medication catheter, and the device further includes a medication bladder configured to contain medication, a bladder pressure applicator to apply pressure to the medication bladder, tubing to provide fluid communication between the medication bladder and the pump, and a bubble detection sensor positioned along a fluid path between the medication bladder and the medication catheter to detect bubbles. In some aspects, the wearable device can include one or more local user interface components such as optical displays, haptic motors, audio speakers, and user input detectors, a housing with an adhesive layer configured to secure the device to a skin surface of the user, and a power component configured to provide power to the device. In some aspects, the device can include one or more needles having dimensions selected based on user-specific parameters such as age, weight, or other physiological characteristics, and the needles may include one or more cannulas configured to administer medication to the user.

In another aspect, a user computing device for food delivery is provided that is configured to obtain food order information from a food order or delivery software application, where the food order information includes food delivery timing and one or more indications of food type, food quantity, food carbohydrate count, food calorie count, or other information usable to determine a medication delivery adjustment. The user computing device can further be configured to communicate at least one of the food order information or an adjustment to medication delivery to a wearable device configured to adjust medication delivery. In some aspects, the user computing device can be a phone and the food order or delivery software application can be an application running on the user computing device. In some aspects, the user computing device can include memory storing executable instructions and communicates the food order information automatically in response to placement of a food delivery order. In some aspects, the user computing device can initiate a request for food order information when placing the food delivery and transmits the food order information via a network, such as the Internet. In some aspects, the application can communicate with the wearable device using a command line interface, graphical user interface, application programming interface call, or another approach to instruct execution of the wearable device. In some aspects, the food order information can include at least delivery timing, food type, and quantity, and medication delivery is determined based on the food order information. In some aspects, the user computing device can communicate the food order information to a sensor or a pump of the wearable device and determines a change in medication delivery based on the food order information and communicates the change to the pump. In some aspects, the user computing device can include one or more computer processors, network interfaces, computer-readable medium drives, datastores, and computer-readable memory systems storing computer program instructions executable by the processors.

In another aspect, a network system is provided that facilitates communication between a user computing device and a wearable device by receiving food order information from the user computing device and transmitting the food order information and/or an adjustment to medication delivery to the wearable device to enable automated medication delivery. In some aspects, the network system can include a publicly accessible network, a private network, or a network configured to be in communication with a plurality of networks. In some aspects, the network system can support Bluetooth Low Energy communication, Wi-Fi communication, near-field communication, or combinations thereof. In some aspects, the network system can include an internal network and a backbone network configured to connect services between the user computing device and the wearable device. In some aspects, the network system can support communication with a cloud-based service and transmits food order information in substantially real time. In some aspects, the network system can be configured to communicate recipes regarding particular food items to the user computing device and to communicate updates of the food order information to the wearable device.

In another aspect, a client application executable on a user computing device is provided that is configured to obtain food order information associated with a food delivery order and communicate the food order information to a wearable device to cause automated medication delivery. In some aspects, the client application can be a food delivery application and sends a request to a food delivery establishment to obtain food order information. In some aspects, the food order information can include nutritional data and ingredient information. In some aspects, the client application can initiate communication automatically upon placement of the food delivery order. In some aspects, the client application can communicate with the wearable device via a network, via Bluetooth, or via Wi-Fi. In some aspects, the client application can communicate with the wearable device without intermediary processing by a cloud server. In some aspects, the client application can provide updated food order information based on delivery status changes and stores historical food order information.

In another aspect, a method for disease management is provided that includes receiving, by a controller of a wearable device, food order information comprising food delivery timing and/or indications of food type, food quantity, food carbohydrate count, food calorie count, or other information usable to determine a medication delivery adjustment, determining a medication adjustment and adjustment timing based on the food order information, and automatically delivering medication to a user using a pump of the wearable device. In some aspects, the method includes measuring at least one physiological measurement from a portion of the user using a sensor. In some aspects, the medication can comprise insulin and automatically delivering the medication includes delivering an insulin bolus. In some aspects, the method can include obtaining sensor output information from a sensor disposed in the wearable device or in a separate wearable device and obtaining physiological measurements based on the sensor output. In some aspects, the method can include obtaining glucose measurements from at least one glucose sensor and determining estimated physiological changes based on the food order information and the physiological measurements. In some aspects, the medication adjustment can be determined based on estimated changes in blood glucose using historical food consumption data, statistical analysis, trend assessment, machine learning, logistic regression, or lookup tables. In some aspects, the method can include calculating a medication dose value, actuating the pump according to a closed-loop medication delivery protocol, storing medication in a medication bladder, applying pressure using a bladder pressure applicator, delivering medication through a catheter, detecting bubbles in a fluid path, conveying medication through tubing, providing user interface outputs, securing the wearable device to the user with an adhesive layer, powering the wearable device, and selecting needles or cannulas based on user-specific physiological parameters.

In another aspect, a method performed by a user computing device for food delivery is provided that includes executing a food delivery application, obtaining food order information from the application, communicating the food order information to a wearable device configured to adjust medication delivery and adjustment timing based on the food order information, and causing automatic delivery of medication according to the adjusted medication delivery and timing. In some aspects, the method can include storing executable instructions in memory and executing the instructions using one or more processors. In some aspects, communication of the food order information can occur automatically in response to placement of a food delivery order and includes initiating a request for food order information. In some aspects, the food order information can be transmitted via a network such as the Internet. In some aspects, communication can use a command line interface, graphical user interface, application programming interface call, or another approach to instruct execution by the wearable device. In some aspects, the method includes determining medication delivery based on the food order information and communicating the food order information to sensors or a pump of the wearable device. In some aspects, the method can include determining and communicating changes in medication delivery and performing operations using processors, network interfaces, computer-readable media, datastores, and memory systems.

In another aspect, a method performed by a network for facilitating communication between a user computing device and a wearable device is provided that includes receiving food order information from the user computing device and transmitting the food order information to the wearable device to execute automated medication delivery. In some aspects, the method can be performed using a publicly accessible network, a private network, or a network in communication with multiple networks. In some aspects, communication can be performed using Bluetooth Low Energy, Wi-Fi, near-field communication, an internal network, or a backbone network connecting services between the user computing device and the wearable device. In some aspects, the method can include supporting communication with a cloud-based service, transmitting food order information in substantially real time, communicating recipes regarding particular food items, and communicating updates of food order information to the wearable device.

In another aspect, a method executed by a client application on a user computing device is provided that includes obtaining food order information associated with a food delivery order and communicating the food order information to a wearable device to cause automated medication delivery. In some aspects, the client application can be a food delivery application and sends a request to a food delivery establishment to obtain food order information. In some aspects, the food order information can include nutritional data and ingredient information. In some aspects, communication can be initiated automatically upon placement of the food delivery order and is performed via a network, Bluetooth, or Wi-Fi. In some aspects, the client application can communicate with the wearable device without intermediary cloud processing, provides updated food order information based on delivery status changes, and stores historical food order information.

### BRIEF DESCRIPTION OF DRAWINGS

Throughout the drawings, reference numbers may be re-used to indicate correspondence between referenced elements. The drawings are provided to illustrate example aspects described herein and are not intended to limit the scope of the disclosure.
FIG. 1 is a block diagram of an illustrative disease management environment, including a physiological monitor system according to some aspects.
FIG. 2 depicts a schematic block diagram illustrating an embodiment of a physiological monitor system according to some aspects.
FIGS. 3A-3B are flow diagrams depicting example routines for food order information and medication delivery to a wearer according to some aspects.
FIG. 4 illustrates an example disease management system that may be part of a disease management environment according to some aspects.
FIG. 5 illustrates an example implementation of a disease management system according to some aspects.
FIG. 6 is a block diagram of an illustrative computing system configured to provide medication delivery to a wearer according to some aspects.

### DETAILED DESCRIPTION

As described above, disease management systems, such as glucose monitors and insulin administration systems, can improve the quality of life of a wearer who requires regular administration of medication and monitoring of various physiological and/or other parameters. The patient's quality of life can be further improved as more components of and/or supporting a disease management system are incorporated into a physiological monitor system.

Various aspects of the disclosure will now be described with regard to certain examples and aspects, which are intended to illustrate but not limit the disclosure. Although aspects of some aspects described in the disclosure will focus, for the purpose of illustration, on particular examples of food delivery with a user computing device, communication between user computing device and physiological monitor, and delivery of medication according to the communication, the examples are illustrative only and are not intended to be limiting. In some aspects, the techniques described herein may be applied to additional or alternative techniques of food delivery with a user computing device, communication between user computing device and physiological monitor, and delivery of medication according to the communication, and the like. Additionally, any feature used in any embodiment described herein may be used in any combination with any other feature or in any other embodiment, without limitation.

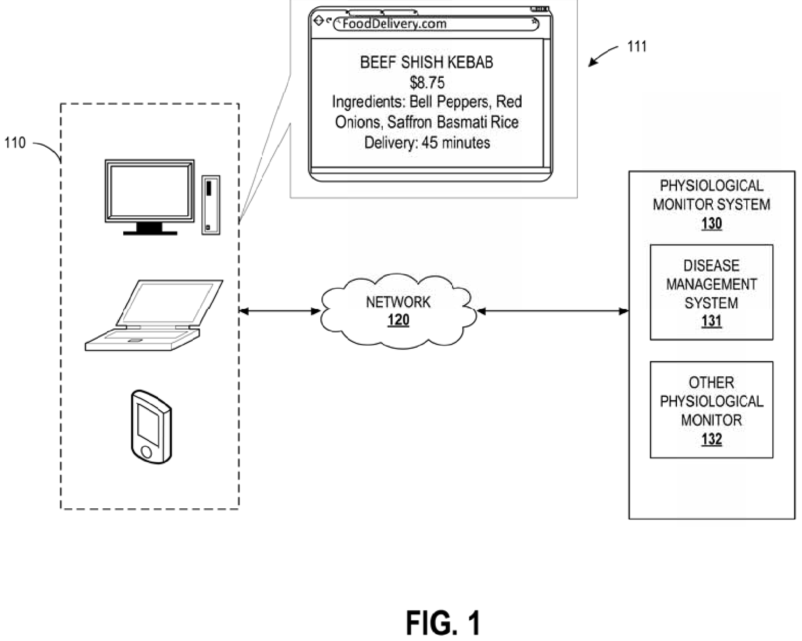

With reference to an illustrative embodiment, FIG. 1 shows an example disease management environment 100 in which aspects of the present disclosure may be implemented. The example disease management environment 100 may provide food order information to a physiological monitor system to control delivery of medication to a wearer. In some examples, the food order information may include timing information (such as, a delivery estimate, indicating imminent food delivery, and/or the like); food type; food quantity; nutritional data about food items (such as, expected blood glucose level in response to consuming the food and/or an estimated change in blood glucose levels); ingredient information and/or recipes; a type, quantity, calorie, total carbohydrate, fiber and/or fat content of food to be delivered; and/or the like. The physiological monitor system may deliver medication (such as, insulin) at a particular time corresponding to the food order information, for example, to provide an amount of insulin in anticipation of blood sugar changes in response to the time information, food type, and food quantity. In some cases, the disease management environment 100 as described herein may provide for more efficient medication delivery by automatically starting the insulin delivery according to the food order information. Thus, increasing the quality of life of the wearer due to the automatic delivery of insulin, rather than administering insulin post-meal. The disease management environment 100 includes user computing devices 110, an example interface 111, a network 120, and a physiological monitor system 130.

The user computing devices 110 may include any devices capable of executing an application for ordering items. For example, the application may allow for ordering food. As shown, the example interface 111 includes an example order for food, which may provide for food order information, as described herein. The user computing devices 110, via the client application, may prompt a command line interface (CLI), graphical user interface (GUI), application programming interface (API) call, or another approach to submit an order for food and/or instruct a physiological monitor to provide medication to a wearer. In some examples, the user computing devices 110 may couple to the physiological monitor system, for example, via the network 120. The user computing devices 110 may be any computing device such as a desktop, laptop or tablet computer, personal computer, wearable computer, server, personal digital assistant (PDA), hybrid PDA/mobile phone, mobile phone, electronic book reader, set-top box, voice command device, camera, digital media player, and the like.

The network 120 may allow the user computing device 110 and the physiological monitor system 130 to communicate information with respect to medication delivery (and/or for the user computing device 110 to place an order for food). The network 120 may allow the user computing device 110 to send a request for food delivery, for example, via a client application executing on the user computing device 110. In some aspects, the network 120 may be a publicly-accessible network of linked networks, possibly operated by various distinct parties, such as the Internet. In some cases, the network 120 may be or include a private network, personal area network, local area network, wide area network, global area network, cable network, satellite network, cellular data network, etc., or a combination thereof, some or all of which may or may not have access to and/or from the Internet. In some cases, the network 120 may include Bluetooth Low Energy (BLE), Wi-Fi, near field communication (NFC), and/or the like. Although FIG. 1 illustrates a single network 120, the illustration is provided for purposes of example only, and is not intended to be limiting, required, or exhaustive. In some aspects, the network 120 may include, or be in communication with, a plurality of networks. For example, the network 120 may include an internal network and a backbone network. The internal network may include a network connecting compute accelerators (or other computing hardware) together. The backbone network may connect services between the components as illustrated in FIG. 1

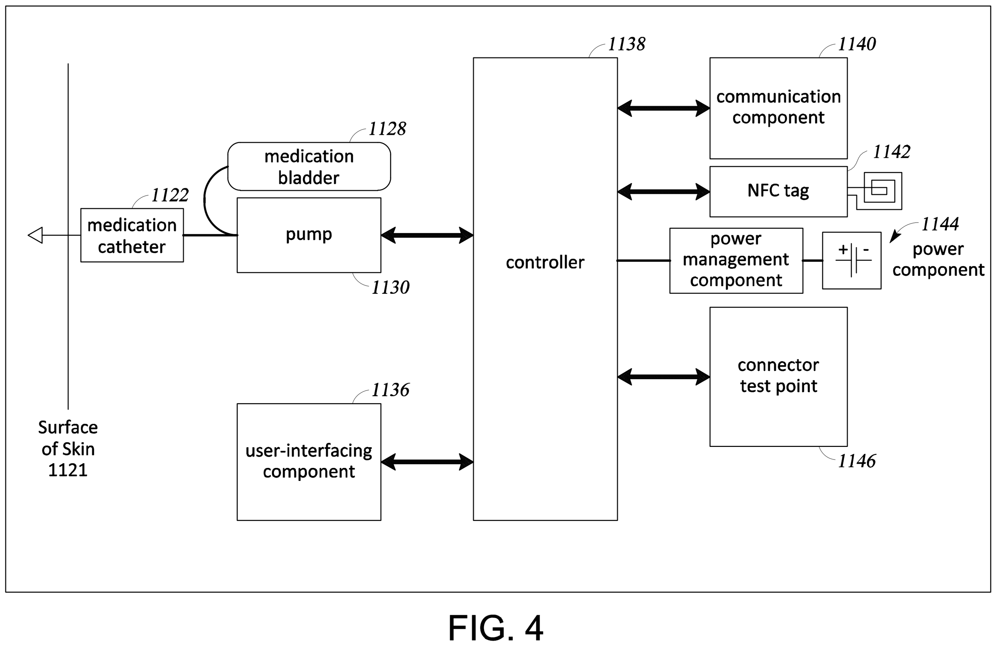

The physiological monitor system 130 may provide for monitoring a wearer's physiological parameters and deliver medication to the wearer. The physiological monitor system 130 may include a disease management system 131 and other physiological monitor 132. The disease management system 131 may provide for detecting changes in a physiological parameter of the wearer and/or delivering medication to the wearer. In some cases, the disease management system 131 may be a glucose monitor with an insulin pump and insulin bolus to provide insulin to the wearer. The disease management system 131 may be as described herein (for example, as described in FIGS. 4-5). In various implementations, the disease management system 131 can include some or all components of a closed loop medication administration system in a self-contained unit. The disease management system 131 may be applied on a patient allowing for ease of installation and removal of the disease management device. However, other applications may also be possible.

In some examples, the disease management system 131 (and components thereof) may be similar or identical to and/or incorporate any of the features described and/or illustrated with respect to any of the devices, assemblies, systems, and/or methods described and/or illustrated in U.S. Application No. 17/161,528, filed January 28, 2021 and titled "METHOD OF OPERATING REDUNDANT STAGGERED DISEASE MANAGEMENT SYSTEMS," the disclosure of which is hereby incorporated by reference in its entirety and is described in Appendix A, and which claims priority to U.S. Provisional Application No. 62/968,107, which was filed on January 30, 2020 and is titled "CLOSED LOOP INSULIN DELIVERY SYSTEM," and also claims priority to U.S. Provisional Application No. 62/978,480, which was filed on February 19, 2020 and is titled "REDUNDANT STAGGERED GLUCOSE SENSOR INSULIN DOSAGE SYSTEM," and also claims priority to U.S. Provisional Application No. 63/015,272, which was filed on April 24, 2020 and is titled "REDUNDANT STAGGERED GLUCOSE SENSOR INSULIN DOSAGE SYSTEM," and also claims priority to U.S. Provisional Application No. 63/044,831, which was filed on June 26, 2020 and is titled "REDUNDANT STAGGERED GLUCOSE SENSOR INSULIN DOSAGE SYSTEM," the disclosures of which are expressly incorporated by reference herein in its entirety for all purposes. Further, the disease management system 131 may be similar or identical to and/or incorporate any of the features described and/or illustrated with respect to any of the devices, assemblies, systems, and/or methods described and/or illustrated in PCT Application No. PCT/US2021/015559, which was filed on January 28, 2021 and is titled "REDUNDANT STAGGERED GLUCOSE SENSOR DISEASE MANAGEMENT SYSTEM," the disclosure of which is hereby incorporated by reference in its entirety and is described in Appendix B.

In some examples, the disease management system 131 may be similar or identical to and/or incorporate any of the features described and/or illustrated with respect to any of the devices, assemblies, systems, and/or methods described and/or illustrated in U.S. Application No. 18/155,978, filed January 18, 2023 and titled "MODULAR WEARABLE DEVICE FOR PATIENT MONITORING AND DRUG ADMINISTRATION," the disclosure of which is hereby incorporated by reference in its entirety and is described in Appendix C, and which claims priority to U.S. Provisional Application No. 63/300,426, which was filed on January 18, 2022 and is titled "MODULAR WEARABLE DEVICE FOR PATIENT MONITORING AND DRUG ADMINISTRATION," the disclosure of which is expressly incorporated by reference herein in its entirety for all purposes.

In some examples, the disease management system 131 may be similar or identical to and/or incorporate any of the features described and/or illustrated with respect to any of the devices, assemblies, systems, and/or methods described and/or illustrated in U.S. Application No. 17/817,613, filed August 4, 2022 and titled "MEDICATION DELIVERY PUMP FOR REDUNDANT STAGGERED GLUCOSE SENSOR INSULIN DOSAGE SYSTEM," the disclosure of which is hereby incorporated by reference in its entirety and is described in Appendix D, and which claims priority to U.S. Provisional Application No. 63/229,305, which was filed on August 4, 2021 and is titled "MEDICATION DELIVERY PUMP FOR REDUNDANT STAGGERED GLUCOSE SENSOR INSULIN DOSAGE SYSTEM," the disclosure of which is expressly incorporated by reference herein in its entirety for all purposes. Further, the disease management system 131 may be similar or identical to and/or incorporate any of the features described and/or illustrated with respect to any of the devices, assemblies, systems, and/or methods described and/or illustrated in PCT Application No. PCT/US2022/039477, which was filed on August 4, 2022 and is titled "MEDICATION DELIVERY PUMP HAVING GLUCOSE SENSOR," the disclosure of which is hereby incorporated by reference in its entirety and is described in Appendix E.

In some examples, the disease management system 131 may be similar or identical to and/or incorporate any of the features described and/or illustrated with respect to any of the devices, assemblies, systems, and/or methods described and/or illustrated in U.S. Application No. 18/605,630, filed March 14, 2024 and titled "MODULAR DISEASE MANAGEMENT DEVICE AND AUTOMATED NEEDLE AND CANNULA INSERTION DEVICE," the disclosure of which is hereby incorporated by reference in its entirety and is described in Appendix F, and which claims priority to U.S. Provisional Application No. 63/490,601, which was filed on March 16, 2023 and is titled "MODULAR DISEASE MANAGEMENT DEVICE AND AUTOMATED NEEDLE AND CANNULA INSERTION DEVICE," the disclosure of which is expressly incorporated by reference herein in its entirety for all purposes. Further, the disease management system 131 may be similar or identical to and/or incorporate any of the features described and/or illustrated with respect to any of the devices, assemblies, systems, and/or methods described and/or illustrated in PCT Application No. PCT/US2024/020096, which was filed on March 15, 2024 and is titled "MODULAR DISEASE MANAGEMENT DEVICE AND AUTOMATED NEEDLE AND CANNULA INSERTION DEVICE," the disclosure of which is hereby incorporated by reference in its entirety and is described in Appendix G.

In some examples, the disease management system 131 may be similar or identical to and/or incorporate any of the features described and/or illustrated with respect to any of the devices, assemblies, systems, and/or methods described and/or illustrated in U.S. Application No. 18/419,408, filed January 22, 2024 and titled "MEDICATION BLADDER FOR MEDICATION STORAGE," the disclosure of which is hereby incorporated by reference in its entirety and is described in Appendix H, and which claims priority to U.S. Provisional Application No. 63/481,365, which was filed on January 24, 2023 and is titled "CONTAINER FOR MEDICATION STORAGE," the disclosure of which is expressly incorporated by reference herein in its entirety for all purposes. Further, the disease management system 131 may be similar or identical to and/or incorporate any of the features described and/or illustrated with respect to any of the devices, assemblies, systems, and/or methods described and/or illustrated in PCT Application No. PCT/US2024/012461, which was filed on January 22, 2024 and is titled "MEDICATION BLADDER FOR MEDICATION STORAGE," the disclosure of which is hereby incorporated by reference in its entirety and is described in Appendix I.

In some examples, the disease management system 131 may be similar or identical to and/or incorporate any of the features described and/or illustrated with respect to any of the devices, assemblies, systems, and/or methods described and/or illustrated in U.S. Provisional Application No. 63/682,899, filed August 14, 2024 and titled "MICROINFUSION PUMP FOR DISPENSING PRECISE VOLUMES," the disclosure of which is hereby incorporated by reference in its entirety and is described in Appendix J.

In some examples, the disease management system 131 may be similar or identical to and/or incorporate any of the features described and/or illustrated with respect to any of the devices, assemblies, systems, and/or methods described and/or illustrated in U.S. Provisional Application No. 63/697,379, filed September 20, 2024 and titled "WEARABLE DEVICE FOR PATIENT MONITORING AND DRUG ADMINISTRATION," the disclosure of which is hereby incorporated by reference in its entirety and is described in Appendix K.

The other physiological monitor 132 may provide for monitoring of physiological features of the wearer different than the disease management system. For example, the other physiological monitor 132 may include one or more sensors for detecting physiological features of the wearer. in some cases, the one or more sensors may include one or more acoustic sensors, one or more electrocardiogram (ECG) sensors, one or more bioimpedance sensors, one or more optical sensors, and one or more other sensors. The acoustic sensors can include piezoelectric transducers or other acoustic transducers for measuring a patient's body sounds (such as breathing and heart sounds), for example, a phonocardiogram (PCG). In one embodiment, the acoustic sensor is placed over the heart or near the heart of a patient to detect heart sounds of the patient. The acoustic sensor can be positioned on the chest, back, neck, side, abdomen, or other area of the body to detect the heart sounds. Heart sounds can include, among others, first and second heart sounds. The first heart sound can correspond to systole, or the contraction of the ventricles and corresponding ejection of blood from the heart. The ECG sensor(s) can include ECG leads or the like for measuring the electrical activity of the heart. The bioimpedance sensor(s) can include electrodes placed on the neck and/or thorax for measuring the impedance of electrical signals in the body. The one or more optical sensors may include a photoplethysmogram (PPG), for example, to measure optical changes along a surface of the wearer's skin from pulse rate. In some examples, the other sensors may include any sensors related to detecting irregular heartbeats. In some cases, the other sensors may include motion sensors, such as, an inertial measurement unit (IMU) or another such motion sensor (for example, an accelerometer or global positioning system (GPS)).

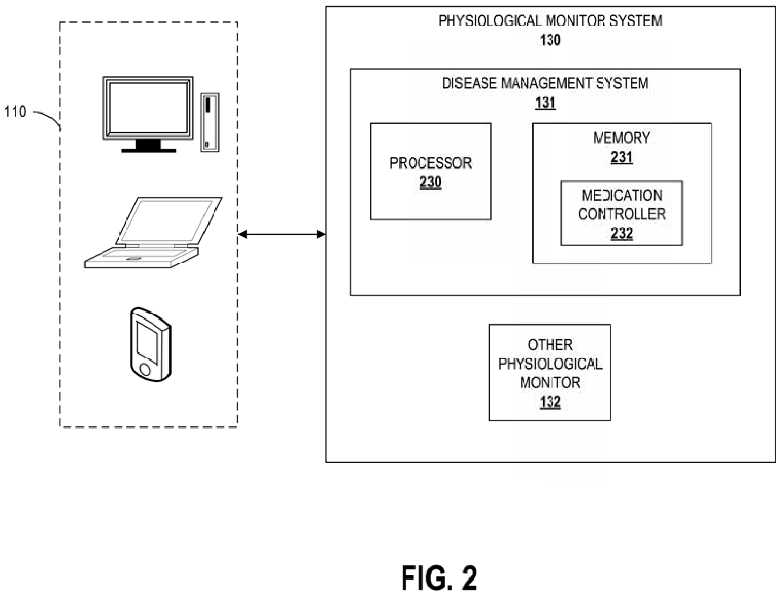

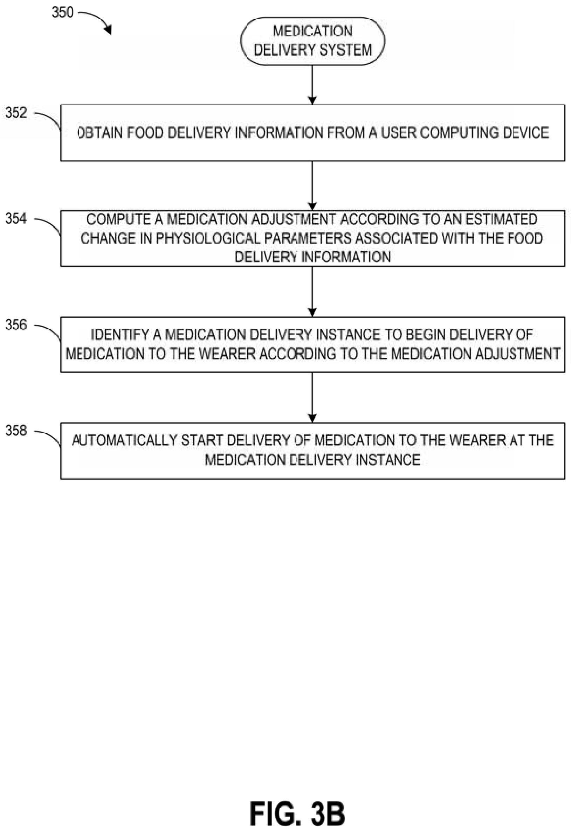

FIG. 2 illustrates an embodiment of a physiological monitor system 130 that can automatically provide medication to a wearer according to order information. The physiological monitor system 130 can implement certain features of the systems as described herein. In particular, the physiological monitor system 130 can obtain the order information associated with, for example, food delivery. The depicted modules can be implemented in hardware and/or in software (for example, as instructions stored in memory 231 executed by one or more processors 230 and/or computing devices). In the depicted embodiment, the physiological monitor system 130 includes a medication delivery system 131, which can be implemented in hardware and/or software. The example medication delivery system 131 includes a processor 230 and a memory 231. The memory 231 may include instructions, executable by the processor 230, to implement a medication controller 232. The medication controller 232 can compute medication delivery measurements and/or timing using the outputs of some or all of the user computing devices 110. In some cases, based at least in part on the order information, the medication controller 232 can deliver medication (such as, insulin) to the wearer. The medication controller 232 may provide medication delivery as described herein (for example, as described in FIGS. 3B and 4-5).

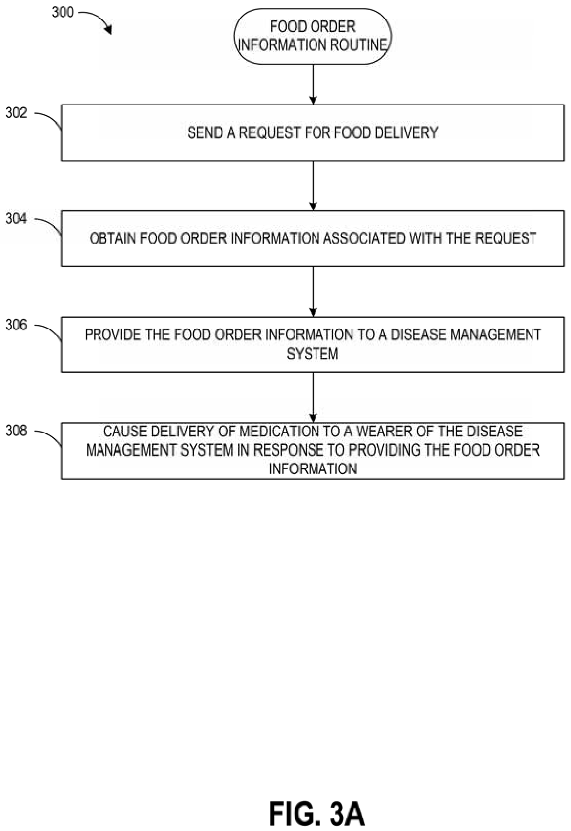

FIG. 3A is a flow diagram of an illustrative routine 300 that may be executed by a user computing device 110 to order food and cause a disease management system to deliver medication to a wearer in response to providing food order information. The routine 300 may begin in response to an event, such as receipt by the user computing device of a request to order food. For example, a user computing device may submit a request including or referencing an application to order food, as described herein.

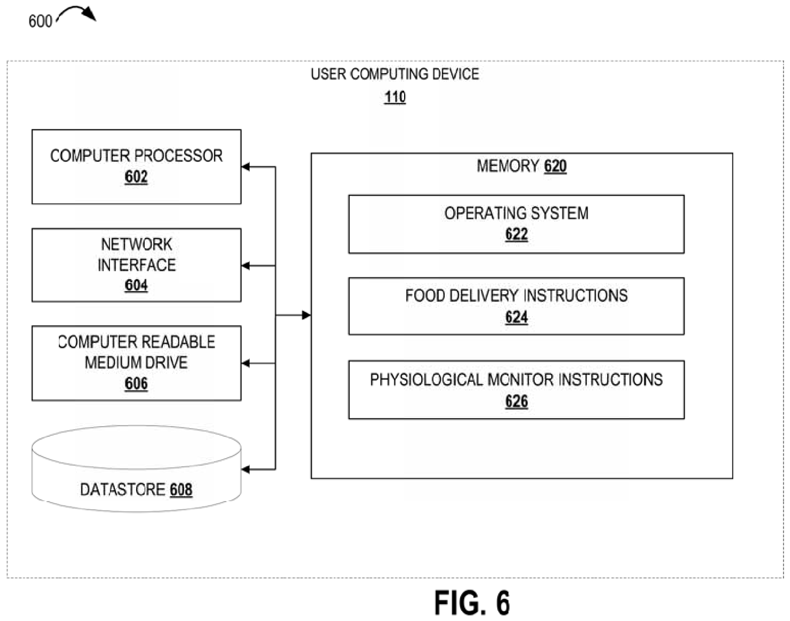

When the routine 300 is initiated, a set of executable program instructions stored on one or more non-transitory computer-readable media (e.g., hard drive, flash memory, removable media, etc.) may be loaded into memory (e.g., random access memory or "RAM") of a serving configuration system, such as the user computing device as shown in FIG. 6, and executed by one or more processors. In some aspects, the routine 300 or portions thereof may be implemented on multiple processors, serially or in parallel.

At step 302, the user computing device sends a request for food delivery. For example, a client application executing on the user computing device may send the request to a food delivery establishment (such as, a restaurant). The user computing device may provide the request via a network (such as, the network 120 in FIG. 1). The request may include an order for food delivery, for example, identifying a type of food, quantity of food, modifications to a food type, and/or the like.

In some examples, the user computing device may send the request for food delivery according to recipes posted on a restaurant's online menu. In some cases, the user computing device sending the request may additionally send a request for the restaurant to provide recipes, ingredients, materials, and/or the like, with respect to selected items. For example, in response to selecting a particular food item, the user computing device may prompt the restaurant (such as, via an online query across a network) to provide recipes regarding the particular food item.

At step 304, the user computing device obtains food order information associated with the request. The food order information may include aspects as disclosed herein (for example, as described in FIG. 1).

At step 306, the user computing device provides the food order information to a disease management system. The user computing device may send the food order information to the disease management system via the network (as described herein, for example, the network 120 in FIG. 1). The disease management system may be as described herein.

At step 308, the user computing device causes delivery of medication to a wearer of the disease management system in response to providing the food order information. The medication may be insulin as part of the disease management system, as described herein. The food order information may include the timing information corresponding to delivery of the food, which may represent a time in which the wearer may consume the food and cause a change in blood sugar.

In the above description of FIG. 3A, any blocks described can include alternate implementations within the scope of the example aspects of the present disclosure in which the blocks can be executed out of order from that shown or discussed, including substantially concurrently or in reverse order, depending upon the functionality involved, as would be understood by those skilled in the art. The various elements, features, and processes described herein may be used independently of one another, or may be combined in various ways. All possible combinations and sub-combinations are intended to fall within the scope of this disclosure.

FIG. 3B is a flow diagram of an illustrative routine 350 that may be executed by a disease management system 131 to provide efficient delivery of medication to a wearer. The routine 350 may begin in response to an event, such as receipt by the disease management system of food order information indicating an expected adjustment of physiological parameters of the wearer. For example, a user computing device may send food order information to the physiological monitor system, which may cause the disease management system to provide delivery of medication, as described herein.

When the routine 300 is initiated, a set of executable program instructions stored on one or more non-transitory computer-readable media (e.g., hard drive, flash memory, removable media, etc.) may be loaded into memory (e.g., random access memory or "RAM") of a serving configuration system, such as the disease management system shown in FIGS. 4-5, and executed by one or more processors. In some aspects, the routine 300 or portions thereof may be implemented on multiple processors, serially or in parallel.

At step 352, the disease management system obtains food order information. For example, the disease management system may obtain the food order information from a user computing device. The food order information may include details as described herein (for example, as described in FIG. 1).

At step 354, the disease management system computes a medication adjustment according to an estimated change in physiological parameters associated with the food order information. The disease management system may, according to a difference between a current measurement of a physiological parameter (such as, blood glucose) and an expected measurement of the physiological parameter (such as, blood glucose), compute the medication adjustment (such as, an insulin adjustment) to deliver to the wearer, for example, to offset an estimated change of the physiological parameter. The disease management system may compute the medication adjustment by performing statistical analysis, trend assessment, and/or another form of computation to compute the medication adjustment. In some cases, the disease management system may apply logistic regression, machine learning techniques, referencing a lookup table, and/or the like, to compute the expected blood glucose levels.

The disease management system may compute the estimated change in the physiological parameters by obtaining a current measurement and expected measurement from a wearer. For example, a current measurement and expected measurement of blood glucose of the wearer. The disease management system may obtain the current measurement by performing a blood glucose measurement on the wearer, for example, by measuring blood glucose content of a sample of fluid from the wearer.

The disease management system may obtain the expected blood glucose level of the wearer by determining a blood glucose level of the wearer in response to consuming the food. The disease management system may determine the expected blood glucose of the wearer based on the food order information and/or historic food consumption data. The historic food consumption data may be a collection of data obtained by the disease management system (for example, stored on the disease management system) representing changes in blood glucose of the wearer in response to consuming various foods, ingredients, drinks, and/or the like. The disease management system may compare the food type from the food order information with the historic food consumption data to obtain the expected blood glucose level. For example, the disease management system may perform statistical analysis, trend assessment, and/or another form of computation to compute the expected blood glucose levels. In some cases, the disease management system may apply logistic regression, machine learning techniques, referencing a lookup table, and/or the like, to compute the expected blood glucose levels.

At step 356, the disease management system identifies a medication delivery instance to begin delivery of medication to the wearer. In some cases, the disease management system may identify the medication delivery instance according to the food order information, expected blood glucose levels, among other factors impacting the medication delivery instance to begin delivery of medication. In some cases, the disease management system may identify a first time period (such as, the current time) and a second time period (such as, the delivery time from the food order information) and identify the medication delivery instance as a third time period between the first time period and/or the second time period (or before, at, or after the first time period and/or the second time period).

At step 358, the disease management system automatically starts delivery of medication to the wearer. In some examples, the disease management system may automatically deliver medication to the wearer at the medication delivery instance. In some cases, the disease management system may receive an input to immediately begin delivery of medication. For example, an input from the wearer (or an input to the disease management system from the user computing system and/or another system). In this way, there may be a manual initiation of medication delivery.

In the above description of FIG. 3B, any blocks described can include alternate implementations within the scope of the example aspects of the present disclosure in which the blocks can be executed out of order from that shown or discussed, including substantially concurrently or in reverse order, depending upon the functionality involved, as would be understood by those skilled in the art. The various elements, features, and processes described herein may be used independently of one another, or may be combined in various ways. All possible combinations and sub-combinations are intended to fall within the scope of this disclosure.

FIG. 4 shows a block diagram of an example disease management system 1101. In some examples, the disease management system 1101 may be part of a disease management environment, such as described above. A disease management system 1101 may be configured to measure one or more physiological parameters of a patient (such as pulse, skin temperature, or other values), measure one or more analytes present in the blood of a patient (such as glucose, lipids, or other analyte) and administer medication (such as insulin, glucagon, or other medication). In some examples, a disease management system 1101 may be configured to communicate with one or more hardware processors that may be external to the disease management system 1101, such as a cloud-based processor or user device. A disease management system 1101 may include an NFC tag to support authentication and pairing with a user device (for example, smart phone or smart watch), Bluetooth communication with additional disease management systems or devices, and Bluetooth communication with a paired user device running an associated control application. To support ease of use and safe interaction with the patient, the system may incorporate user input through a tap-detecting accelerometer and provide feedback via an audio speaker, haptic vibration, and/or optical indicators. The system may operate on battery power and support both shelf- life and reliable operation once applied to the patient. Battery life may be managed through control of several planned levels of sleep and power consumption. To support this reliability, a controller can monitor several system-health parameters, and monitor temperatures of the included medication, and ambient temperature for the life of the device.

As illustrated in FIG. 4, a controller 1138 of the disease management system 1101 may be configured to communicate and control one or more components of the disease management system 1101. The controller 1138 may include one or more hardware processors, such as a printed circuit board (PCB) or the like. The controller 1138 may be configured to communicate with peripheral devices or components to support the accurate measurement of physiological parameters and blood analytes, such as patient pulse, temperature, and blood glucose, using detector electronics. The controller 1138 may subsequently calculate dose or receive a calculated dose value and administer medication, such as insulin, by actuation of an actuated pump. The controller 1138 may record device activity and transfer the recorded data to non-volatile secure memory space. At the end of the life of a device or system, the controller can be configured to lock operation, and create a data recovery module to permit authenticated access to the recorded data if needed.

A disease management system 1101 may optionally include an analyte sensor 1120. The analyte sensor 1120 may be configured to detect analytes in the patient's blood. For example, an analyte sensor 1120 can include a glucose sensing probe configured to pierce the surface of the skin 1121. In some examples, a disease management system 1101 may include a plurality of analyte sensors 1120 to detect one or more analytes. In some examples, an analyte sensor 1120 may be configured to detect a plurality of analytes. Sensed analytes may include, but are not limited to, glucose, insulin, and other analytes. An analyte sensor 1120 may be configured to communicate with an optional additional component such as an analyte detector 1126. The analyte detector 1126 may be configured to receive a signal of one or more analyte sensors 1120 in order to measure one or more analytes in the blood of the patient. The analyte detector 1126 may be configured to communicate with the controller 1138. For example, the analyte detector 1126 may be configured to, for example, send analyte values to the controller 1138 and receive control signals from the controller.

A disease management system 1101 may include a medication catheter 1122. The medication catheter 1122 may be configured to administer medication, including, but not limited to insulin, to the patient. The medication catheter 1122 may receive medication from a medication bladder 1128 configured to contain medication to be administered. The medication bladder 1128 may be configured to contain medication for a prolonged period, such as 1 day, 3 days, 6 days, or more. The medication bladder 1128 may be configured to contain certain medication types, such as insulin. In some examples, a disease management system 1101 may include a plurality of medication bladders 1128 for one or more reservoirs of the same or different medications. In some examples, a disease management system 1101 may be configured to mix medications from medication bladders 1128 prior to administration to the patient. A pump 1130 may be configured to cause medication to be administered from the bladder 1128 to the patient through the insulin catheter 1122. A pump 1130 may include, but is not limited to, a pump such as described herein. The disease management system 1101 may include a bubble detection sensor 1132 that can detect bubbles in the liquid drug within any or multiple of fluid lines connecting the medication bladder 1128, pump 1130, and medication catheter 1122.

A disease management system 1101 may optionally include a physiological sensor 1124. The physiological sensor 1124 may include a pulse rate sensor, temperature sensor, pulse oximeter, the like or a combination thereof. In some examples, a disease management system 1101 may be configured to include a plurality of physiological sensors. The physiological sensor 1124 may be configured to communicate with an optional additional component such as a physiological detector 1134. The physiological detector 1134 may be configured to receive a signals of the physiological sensor 1124. The physiological detector 1134 may be configured to measure or determine and communicate a physiological value from the signal. The physiological detector 1134 may be configured to communicate with the controller 1138. For example, the physiological detector 1134 may be configured to, for example, send measured physiological values to the controller 1138 and receive control signals from the controller.

A disease management system 1101 may include one or more local user interfacing components 1136. For example, a local user interfacing component 1136 may include, but is not limited to one or more optical displays, haptic motors, audio speakers, and user input detectors. In some examples, an optical display may include an LED light configured to display a plurality of colors. In some examples, an optical display may include a digital display of information associated with the disease management system 1101, including, but not limited to, device status, medication status, patient status, measured analyte or physiological values, the like or a combination thereof. In some examples, a user input detector may include an inertial measurement unit, tap detector, touch display, or other component configured to accept and receive user input. In some examples, audio speakers may be configured to communicate audible alarms related to device status, medication status user status, the like or a combination thereof. A controller 1138 may be configured to communicate with the one or more local interfacing components 1136 by, for example, receiving user input from the one or more user input components or sending control signals to, for example, activate a haptic motor, generate an output to the optical display, generate an audible output, or otherwise control one or more of the local user interfacing components 1136.

A disease management system 1101 may include one or more communication components 1140. A communication component 1140 can include but is not limited to one or more radios configured to emit Bluetooth, cellular, Wi-Fi, or other wireless signals. In some examples, a communication component 1140 can include a port for a wired connection. Additionally, a disease management system 1101 may include an NFC tag 1142 to facilitate in communicating with one or more hardware processors. The one or more communication components 1140 and NFC tag 1142 may be configured to communicate with the controller 1138 in order to send and/or receive information associated with the disease management system 1101. For example, a controller 1138 may communicate medication information and measured values through the one or more communication components 1140 to an external device. Additionally, the controller 1138 may receive instructions associated with measurement sampling rates, medication delivery, or other information associated with operation of the management system 1101 through the one or more communication components 1140 from one or more external devices.

A disease management system 1101 may include one or more power components 1144. The power components may include but are not limited to one or more batteries and power management components, such as a voltage regulator. Power from the one or more power components 1144 may be accessed by the controller and/or other components of the disease management system 1101 to operate the disease management system 1101.

A disease management system 1101 may have one or more power and sleep modes to help regulate power usage. For example, a disease management system 1101 may have a sleep mode. The sleep mode may be a very low power mode with minimal functions, such as the RTC (or real time clock) and alarms to wake the system and take a temperature measurement of the system, or the like. In another example, a disease management system 1101 may include a measure temperature mode which may correspond to a low power mode with reduced functions. The measure temperature mode may be triggered by the RTC where the system is configured to take a temperature measurement, save the value, and return the system to a sleep mode. In another example, a disease management system 1101 may include a wake-up mode. The wake-up mode may be triggered by an NFC device and allow the system to pair with an external device with, for example, Bluetooth. If a pairing event does not occur, the system may return to sleep mode. In another example, a disease management system 1101 may include a pairing mode. The pairing mode may be triggered by an NFC device. When a controlling application is recognized, the system may proceed to pair with the application and set the system to an on condition and communicate to the cloud or other external device to establish initial data movement. In another example, a disease management system 1101 may include a rest mode where the system is configured to enter a lower power mode between measurements. In another example, a disease management system 1101 may include a data acquisition mode where the system is configured to enter a medium power mode where data acquisition takes place. In another example, a disease management system 1101 may include a parameter calculation mode where the system is configured to enter a medium power mode where parameter calculations, such as a blood glucose calculation, are performed and data is communicated to an external device and/or the cloud. In another example, a disease management system 1101 may include a pump mode where the system is configured to enter a higher power mode where the pump draws power to deliver medication to the patient.

A disease management system 1101 may include one or more connector test points 1146. The connecter test points may be configured to aid in programming, debugging, testing or other accessing of the disease management system 1101. In some examples, connector test points 1146 may include, for example, a GPIO spare, UART receiver or transmitter, the like or a combination thereof.

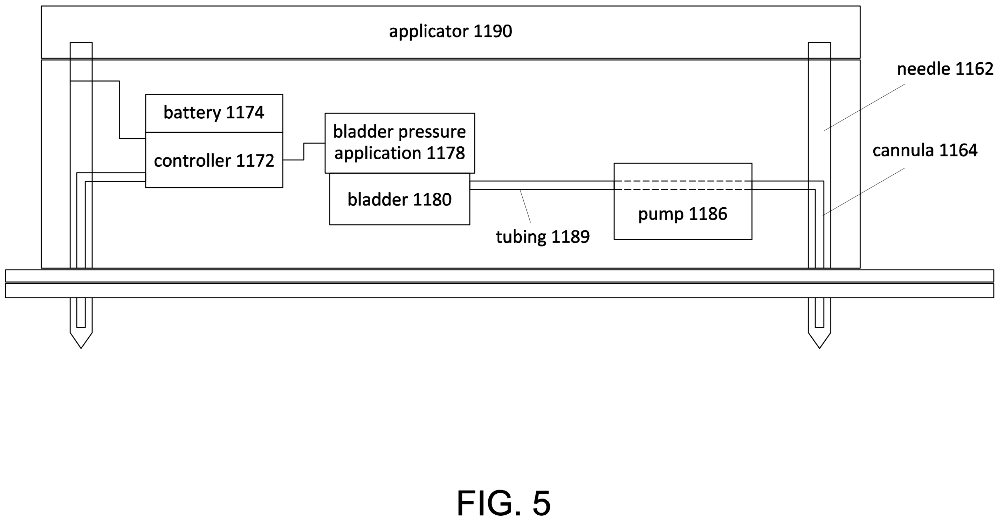

FIG. 5 illustrates an example implementation of a disease management system 1103 and applicator 1190 for applying a disease management system 1103 to a patient. Disease management system 1103 can include any one or more of the features discussed above with respect to the disease management system 1101 in addition to the features described below. In the illustrated example, an applicator 1190 may be configured to mate with the disease management system 1103. In some examples, an applicator 1190 may include a safety button 1192 for release or other interaction with the applicator 1190. In the illustrated example, a disease management system 1103 may include one or more LEDs 1160 that may be configured to output information using one or more of color, frequency, and length of display. In some examples, the disease management system 1103 may include a buzzer 1176, haptic actuator 1170, or other feedback mechanism, such as a speaker to output information to the patient, such as an alarm. In some examples, a disease management system 1103 may include a battery 1174, controller 1172. In some examples, a disease management system 1103 may include aspects of a medication administration system, such as a bladder 1180, a bladder pressure applicator 1178 to provide pressure on the bladder (such as a component of a pump), actuator 1182, pump gears 1184, and a pump 1186. In some examples, a disease management system 1103 may include one or more tubing 1188 configured to allow for fluid communication between the bladder 1180, pump 1186, and cannula 1164. In some examples, a disease management system 1103 may include one or more needles 1158 that may include one or more analyte sensors (such as a glucose sensor) 1156. In some examples, a disease management system 1103 may include one or more needles 1162 that may include one or more cannulas 1164 configured to administer medication to the patient. In some examples, a disease management system 1103 may include an air bubble sensor 1152 configured to detect the presence of air bubbles in the medication prior to delivery to the patient. In some examples, a glucose control system 1103 may include one or more physiological sensors 1154, such as a non-invasive physiological sensor including but not limited to a pulse sensor. In some examples, the disease management system 1103 may include a base plate 1106 and an adhesive layer 1168 below the base plate 1106 to provide adhesion of the disease management system 1103 to the patient's skin. As described below, a housing of the disease management system 1103 may consist of a combination of flexible and rigid material so as to both provide support for the components of the disease management system 1103 and allow conforming, at least in part, of the disease management system 1103 to the skin of the patient.

The adhesive layer 1168 may be configured to provide adhesion for a prolonged period. For example, the adhesive layer 1168 may be configured to adhere the disease management system 1103 to the skin of a patient for a period of 1 day, 3 days, 6 days, or more or fewer days or hours. In some examples, the adhesive layer may be configured to have an adhesive force sufficient to prevent accidental removal or movement of the disease management system 1103 during the intended period of use of the disease management system 1103. In some examples, the adhesive layer 1168 may be a single layer of adhesive across at least a portion of a surface the disease management system 1103 that is configured to interface with the patient. In some examples, the adhesive layer 1168 may include a plurality of adhesive areas on a surface of the disease management system 1103 that is configured to interface with the patient. In some examples, the adhesive layer 1168 may be configured to be breathable, adhere to the patient's skin after wetting by humidity or liquids such as tap water, saltwater, and chlorinated water. A thickness of the adhesive may be, for example, in a range of 0.1 to 0.5 mm or in a range of more or less thickness.

In some examples, a needle 1158, 1162 may be inserted at different depths based on a patient age, weight, or other parameter. For example, a depth of insertion of a medication cannula may be approximately 3 mm for 7 to 12 year olds. In another example, a depth of insertion of a medication cannula may be approximately 4 mm for 13 year olds and older. In another example, a depth of insertion of a medication needle may be approximately 4 to 4.5 mm for 7 to 12 year olds. In another example, a depth of insertion of a medication needle may be approximately 5 to 5.5 mm for 13 year olds and older. In another example, a depth of insertion of an analyte sensor may be approximately 3 mm for 7 to 12 year olds. In another example, a depth of insertion of an analyte sensor may be approximately 4 mm for 13 year olds and older. In another example, a depth of insertion for a needle associated with an analyte sensor may be approximately 4 to 4.5 mm for 7 to 12 year olds. In another example, a depth of insertion for a needle associated with an analyte sensor may be approximately 5 to 5.5 mm for 13 year olds and older. However, other values or ranges for any of the inserted components are also possible.

FIG. 6 illustrates various components of an example computing device 600 configured to implement various functionality of the user computing device 110. In some aspects, as shown, the user computing device 110 may include: one or more computer processors 602, such as physical central processing units ("CPUs"); one or more network interfaces 604, such as a network interface cards ("NICs"); one or more computer-readable medium drives 606, such as a high density disk ("HDDs"), solid state drives ("SSDs"), flash drives, and/or other persistent non-transitory computer-readable media; one or more datastore 608, such as physical storage and/or remote storage, and/or other data storage components; and one or more computer-readable memories 620, such as random access memory ("RAM") and/or other volatile non-transitory computer-readable media.

The computer-readable memory 620 may include computer program instructions that one or more computer processors 602 execute in order to implement one or more aspects. The computer-readable memory 620 can store an operating system 622 that provides computer program instructions for use by the computer processor(s) 602 in the general administration and operation of the user computing device 110.

In some aspects, the computer-readable memory 620 can further include computer program instructions and other information for implementing aspects of the present disclosure. For example, the computer-readable memory 620 may include food delivery instructions 624 for sending a request for a food delivery order, as described herein. As another example, the computer-readable memory 620 may include physiological monitor instructions 626 for providing food order information to a physiological monitor system, as described herein.

When a routine is initiated, a corresponding set of executable program instructions stored on a computer-readable medium drive 606 may be loaded into computer-readable memory 620 and executed by one or more computer processors 602. In some aspects, a routine-or portions thereof-may be implemented on multiple computing devices and/or multiple processors, serially or in parallel.

### Terminology

Many variations and modifications may be made to the above-described implementations, the elements of which are to be understood as being among other acceptable examples. All such modifications and variations are intended to be included herein within the scope of this disclosure. The foregoing description details certain implementations. It will be appreciated, however, that no matter how detailed the foregoing appears in text, the systems and methods can be practiced in many ways. As is also stated above, it should be noted that the use of particular terminology when describing certain features or aspects of the systems and methods should not be taken to imply that the terminology is being re-defined herein to be restricted to including any specific characteristics of the features or aspects of the systems and methods with which that terminology is associated.

Conditional language, such as, among others, "can," "could," "might," or "may," unless specifically stated otherwise, or otherwise understood within the context as used, is generally intended to convey that certain implementations include, while other implementations do not include, certain features, elements, and/or steps. Thus, such conditional language is not generally intended to imply that features, elements and/or steps are in any way required for one or more implementations or that one or more implementations necessarily include logic for deciding, with or without user input or prompting, whether these features, elements and/or steps are included or are to be performed in any particular implementation.

The term "substantially" when used in conjunction with the term "real-time" forms a phrase that will be readily understood by a person of ordinary skill in the art. For example, it is readily understood that such language will include speeds in which no or little delay or waiting is discernible, or where such delay is sufficiently short so as not to be disruptive, irritating, or otherwise vexing to a user.

Conjunctive language such as the phrase "at least one of X, Y, and Z," or "at least one of X, Y, or Z," unless specifically stated otherwise, is to be understood with the context as used in general to convey that an item, term, and/or the like may be either X, Y, or Z, or a combination thereof. For example, the term "or" is used in its inclusive sense (and not in its exclusive sense) so that when used, for example, to connect a list of elements, the term "or" means one, some, or all of the elements in the list. Thus, such conjunctive language is not generally intended to imply that certain implementations require at least one of X, at least one of Y, and at least one of Z to each be present.

The term "a" as used herein should be given an inclusive rather than exclusive interpretation. For example, unless specifically noted, the term "a" should not be understood to mean "exactly one" or "one and only one"; instead, the term "a" means "one or more" or "at least one," whether used in the claims or elsewhere in the specification and regardless of uses of quantifiers such as "at least one," "one or more," or "a plurality" elsewhere in the claims or specification.

The term "comprising" as used herein should be given an inclusive rather than exclusive interpretation. For example, a general-purpose computer comprising one or more processors should not be interpreted as excluding other computer components, and may possibly include such components as memory, input/output devices, and/or network interfaces, among others.

While the above detailed description has shown, described, and pointed out novel features as applied to various implementations, it may be understood that various omissions, substitutions, and changes in the form and details of the devices or processes illustrated may be made without departing from the spirit of the disclosure. As may be recognized, certain implementations of the inventions described herein may be embodied within a form that does not provide all of the features and benefits set forth herein, as some features may be used or practiced separately from others. The scope of certain inventions disclosed herein is indicated by the appended claims rather than by the foregoing description. All changes which come within the meaning and range of equivalency of the claims are to be embraced within their scope.

## Claims

1. A wearable device, comprising:
a pump configured to deliver a medication to a user;
a controller including one or more processors storing instructions in a memory, wherein in response to execution of the instructions, the controller is configured to:
receive food order information including at least one of food delivery timing and an indication of one or more of a food type, food quantity, food carbohydrate count, food calorie count, or any other indication of the food order information useable to determine a medication delivery adjustment;
determine a medication adjustment and adjustment timing based on the food order information; and
automatically deliver the medication with the pump based on the medication adjustment.

2. The device of claim 1, wherein the medication comprises insulin and the medication delivery comprises an insulin bolus.

3. The device of claim 1, further comprising at least one sensor disposed in the wearable device or in a separate wearable device, wherein the wearable device is configured to obtain a physiological measurement from the sensor.

4. The device of claim 1, further comprising at least one glucose sensor configured to provide glucose measurements to the controller.

5. The device of claim 1, wherein the controller determines an estimated physiological change based on the food order information and at least one physiological measurement.

6. The device of claim 1, wherein the controller is configured to compute the medication adjustment based on an estimated change in blood glucose associated with the food order information.

7. The device of claim 6, wherein the estimated change in blood glucose is determined using at least one of historical food consumption data associated with the user, statistical analysis, trend assessment, machine learning, logistic regression, or a lookup table.

8. The device of claim 1, wherein the food order information further includes ingredient information.

9. The device of claim 1, wherein the controller calculates a medication dose value and administers the medication by actuation of the pump according to a closed-loop medication delivery protocol.

10. The device of claim 1, wherein the pump is fluidly coupled to a medication bladder and a medication catheter.

11. The device of claim 10, further comprising a medication bladder configured to contain medication to be administered and a bladder pressure applicator configured to apply pressure to the medication bladder.

12. The device of claim 10, further comprising a bubble detection sensor positioned along a fluid path between the medication bladder and the medication catheter and configured to detect bubbles within the fluid path.

13. The device of claim 1, further comprising tubing that places medication in a medication bladder in fluid communication with the pump.

14. The device of claim 1, further comprising one or more local user interfacing components comprising at least one of an optical display, haptic motor, audio speaker, or user input detector.

15. The device of claim 1, further comprising a housing including an adhesive layer configured to secure the wearable device to a skin surface of the user and a power component configured to supply power to the wearable device.

16. The device of claim 1, wherein the device comprises one or more needles having dimensions selected based on at least one user-specific physiological parameter including age or weight.

17. A client application, executable on a user computing device, the application being configured to:
obtain food order information associated with a food delivery order; and
communicate the food order information to a wearable device to cause automated medication delivery.

18. The client application of claim 17, wherein the client application comprises a food delivery application that sends a request to a food delivery establishment for the food order information and automatically initiates communication with the wearable device upon placement of the food delivery order.

19. The client application of claim 17, wherein the client application communicates with the wearable device without intermediary processing by a cloud server or via at least one of a network, Bluetooth, or Wi-Fi.

20. The client application of claim 17, wherein the food order information includes nutritional data and ingredient information, the client application provides updated food order information based on delivery status changes, and the client application stores historical food order information.
